# EUROPÄISCHE PATENTSCHRIFT

(11) **EP 1 339 714 B1**
(45) Veröffentlichungstag und Bekanntmachung des Hinweises auf die Patenterteilung: **18.10.2006**
(21) Anmeldenummer: 01989460.9
(22) Anmeldetag: 12.11.2001
(51) Int. Cl.: C07D 471/04, A61K 31/437, A61P 9/00, A61P 15/00, C07D 231/00, C07D 221/00

(54) **NEUE SULFONAMID-SUBSTITUIERTE PYRAZOLOPYRIDINDERIVATE**
NOVEL SULFONAMIDE-SUBSTITUTED PYRAZOLOPYRIDINE DERIVATIVES
NOUVEAUX DERIVES DE PYRAZOLOPYRIDINE SUBSTITUES PAR SULFONAMIDE

(30) Priorität: 22.11.2000 DE 10057754
(43) Veröffentlichungstag der Anmeldung: 03.09.2003
(73) Patentinhaber: Bayer HealthCare AG, 51368 Leverkusen (DE)
(72) Erfinder: STASCH, Johannes-Peter, 42651 Solingen (DE); FEURER, Achim, 69259 Wilhelmsfeld (DE); WEIGAND, Stefan, 42115 Wuppertal (DE); STAHL, Elke, 51467 Bergisch Gladbach (DE); FLUBACHER, Dietmar, 79110 Freiburg (DE); ALONSO-ALIJA, Cristina, 42781 Haan (DE); WUNDER, Frank, 42117 Wuppertal (DE); LANG, Dieter, 42553 Velbert (DE); DEMBOWSKY, Klaus, Boston, MA 02116 (US); STRAUB, Alexander, 42117 Wuppertal (DE); PERZBORN, Elisabeth, 42327 Wuppertal (DE)
(86) Internationale Anmeldenummer: PCT/EP2001/013064
(87) Internationale Veröffentlichungsnummer: WO 2002/042302

(56) Entgegenhaltungen:
- WO-A-00/06567
- WO-A-00/06568
- WO-A-00/06569

## Beschreibung

Die vorliegende Erfindung betrifft neue chemische Verbindungen, welche die lösliche Guanylatcyclase stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffinonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Härns anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 98/16223).

Weiterhin sind in der WO 98/16507, WO 98/23619, WO 00/06567, WO 00/06568, WO 00/06569 und WO 00/21954 Pyrazolopyridinderivate als Stimulatoren der löslichen Guanylatcyclase beschrieben. In diesen Patentanmeldungen sind auch Pyrazolopyridine beschrieben, welche einen Pyrimidinrest in 3-Position aufweisen. Derartige Verbindungen weisen eine sehr hohe in vitro Aktivität bezüglich der Stimulation der löslichen Guanylatcyclase auf Allerdings zeigte es sich, dass diese Verbindungen hinsichtlich ihrer in vivo-Eigenschaften wie beispielsweise ihrem Verhalten in der Leber, ihrem pharmakokinetischen Verhalten, ihrer Dosis-Wirkungsbeziehung oder ihrem Metabolisierungsweg einige Nachteile aufweisen.

Es war daher die Aufgabe der vorliegenden Erfindung, weitere Pyrazolopyridin derivate bereitzustellen, welche als Stimulatoren der löslichen Guanylatcyclase wirken, aber nicht die vorstehend aufgeführten Nachteile der Verbindungen aus dem Stand der Technik aufweisen.

Diese Aufgabe wird gemäß der vorliegenden Erfindungen durch Verbindungen gemäß Anspruch 1 gelöst. Diese neuen Pyrazolopyridinderivate zeichnen sich durch einen Pyrimidinrest in 3-Position aus, der ein bestimmtes Substitutionsmuster aufweist, nämlich einen Sulfonamidrest in 5-Position des Pyrimidinrings sowie eine oder zwei Aminogruppen in 4- und gegebenenfalls 6-Position des Pyrimidinrings.

Im einzelnen betrifft die vorliegende Erfindung Verbindungen der Formel (I) 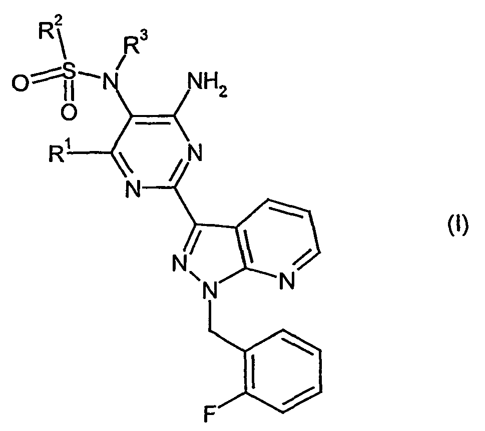 worin
- R¹: für H, Cl oder NH₂ steht;
- R² und R³: zusammen mit den Heteroatomen, an welche sie gebunden sind, einen fünf- bis siebengliedrigen Heterocyclus bilden, der gesättigt oder teilweise ungesättigt sein kann, gegebenenfalls ein oder mehrere weitere Heteroatome aus der Gruppe N, O, S enthalten kann und gegebenenfalls substituiert sein kann;
sowie Salze, Isomere und Hydrate davon.

Bevorzugt sind gemäß der vorliegenden Erfindung Verbindungen der Formel (I), bei denen
- R¹: für H, Cl oder NH₂ steht;
- R² und R³: zusammen mit den Heteroatomen, an welche sie gebunden sind, einen fünf- bis siebengliedrigen Heterocyclus bilden, der gesättigt oder teilweise ungesättigt sein kann, gegebenenfalls ein oder mehrere weitere Heteroatome aus der Gruppe N, O, S enthalten kann;
sowie Salze, Isomere und Hydrate davon.

Besonders bevorzugt sind hierbei Verbindungen der Formel (I), bei denen
- R¹: für H, Cl oder NH₂ steht;
- R² und R³: zusammen mit den Heteroatomen, an welche sie gebunden sind, einen gesättigten fünf- bis siebengliedrigen Heterocyclus bilden;
sowie Salze, Isomere und Hydrate davon.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch chromatographische Trennung, in die stereoisomer einheitlichen Bestandteile trennen. In den erfindungsgemäßen Verbindungen vorhandene Doppelbindungen können in der cis- oder trans- Konfiguration (Z- oder E-Form) vorliegen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Weiterhin können die erfindungsgemäßen Verbindungen in Form ihrer Hydrate vorkommen, wobei die Zahl der an das Molekül gebundenen Wassermoleküle von der jeweiligen erfindungsgemäßen Verbindung abhängt.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.
Alkylen steht im allgemeinen für eine geradkettige oder verzweigte Kohlenwasserstoffbrücke mit 1 bis 20 Kohlenstoffatomen. Beispielsweise seien Methylen, Ethylen, Propylen, α-Methylethylen, β-Methylethylen, α-Ethylethylen, β-Ethylethylen, Butylen, α-Methylpropylen, β-Methylpropylen, γ-Methylpropylen, α-Ethylpropylen, β-Ethylpropylen, γ-Ethylpropylen, Pentylen, Hexylen, Heptylen, Octylen, Nonylen, Decylen, Dodeylen und Eicosylen genannt.
Alkenyl steht im allgemeinen für einen geradkettigen oder verzweigten Kohlenwasserstoffrest mit 2 bis 20 Kohlenstoffatomen und einer oder mehreren, bevorzugt mit einer oder zwei Doppelbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl, Isooctenyl genannt.
Alkinyl steht im allgemeinen für einen geradkettigen oder verzweigten Kohlenwasserstoffrest mit 2 bis 20 Kohlenstoffatomen und einer oder mehreren, bevorzugt mit einer oder zwei Dreifachbindungen. Beispielsweise seien Ethinyl, 2-Butinyl, 2-Pentinyl und 2-Hexinyl benannt.
Acyl steht im allgemeinen für geradkettiges oder verzweigtes Niedrigalkyl mit 1 bis 9 Kohlenstoffatomen, das über eine Carbonylgruppe gebunden ist. Beispielsweise seien genannt: Acetyl, Ethylcarbonyl, Propylcarbonyl, Isopropylcarbonyl, Butylcarbonyl und Isobutylcarbonyl.
Alkoxy steht im allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.
Alkoxyalkyl steht im allgemeinen für einen Alkylrest mit bis zu 8 Kohlenstoffatomen, der durch einen Alkoxyrest mit bis zu 8 Kohlenstoffatomen substituiert ist.
Alkoxycarbonyl kann beispielsweise durch die Formel 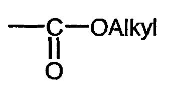 dargestellt werden.
Alkyl steht hierbei im allgemeinen für einen geradkettigen oder verzweigten Kohlenwasserstoffrest mit 1 bis 13 Kohlenstoffatomen. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.
Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Cycloalkoxy steht im Rahmen der Erfindung für einen Alkoxyrest, dessen Kohlenwasserstoffrest ein Cycloalkylrest ist. Der Cycloalkylrest hat im allgemeinen bis zu 8 Kohlenstoffatome. Als Beispiele seien genannt: Cyclopropyloxy und Cyclohexyloxy. Die Begriffe "Cycloalkoxy" und "Cycloalkyloxy" werden synonym verwendet.
Aryl steht im allgemeinen für einen aromatischen Rest mit 6 bis 10 Kohlenstoffatomen. Bevorzugte Arylreste sind Phenyl und Naphthyl.
Halogen steht im Rahmen der Erfindung für Fluor, Chlor, Brom und Iod.
Heterocyclus steht im Rahmen der Erfindung im allgemeinen für einen gesättigten, ungesättigten oder aromatischen 3- bis 10-gliedrigen, beispielsweise 5- oder 6-gliedrigen Heterocyclus, der bis zu 3 Heteroatome aus der Reihe S, N und/oder O enthalten kann und der im Fall eines Stickstoffatoms auch über dieses gebunden sein kann. Beispielsweise seien genannt: Oxadiazolyl, Thiadiazolyl, Pyrazolyl, Pyridyl, Pyrimidinyl, Pyridazinyl, Pyrazinyl, Thienyl, Furyl, Pyrrolyl, Pyrrolidinyl, Piperazinyl, Tetrahydropyranyl, Tetrahydrofuranyl, 1,2,3 Triazolyl, Thiazolyl, Oxazolyl, Imidazolyl, Morpholinyl oder Piperidyl. Bevorzugt sind Thiazolyl, Furyl, Oxazolyl, Pyrazolyl, Triazolyl, Pyridyl, Pyrimidinyl, Pyridazinyl und Tetrahydropyranyl. Der Begriff "Heteroaryl" (bzw. "Hetaryl") steht für einen aromatischen heterocyclischen Rest.

Die erfindungsgemäßen Verbindungen der Formel (I) können hergestellt werden durch die Umsetzung der Verbindung der Formel (II) 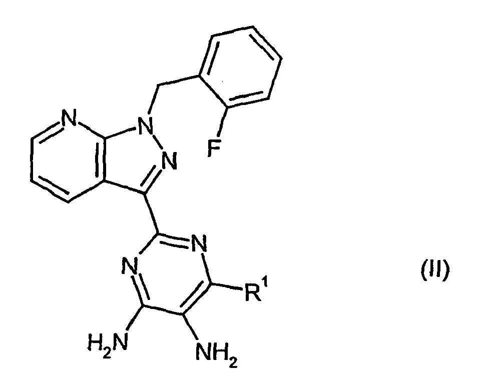 wobei R¹ wie vorstehend definiert ist;
mit einer Verbindung der Formel X-L-SO₂X
wobei
- X: für eine durch eine Aminogruppe substituierbare Abgangsgruppe wie beispielsweise Halogen steht;
- L: für eine Alkandiylgruppe oder eine Alkendiylgruppe mit jeweils 3 bis 5 Kohlenstoffatomen steht, wobei ein oder mehrere Kohlenstoffatome durch ein oder mehrere Heteroatome aus der Gruppe N, O, S ersetzt sein können, und wobei die Gruppe gegebenenfalls substituiert sein kann;
in Gegenwart einer organischen Base bei Raumtemperatur und anschließender Umsetzung mit einer Base in einem organischen Lösungsmittel unter Erhitzen.

Die Ausgangsverbindung der Formel (IIa) 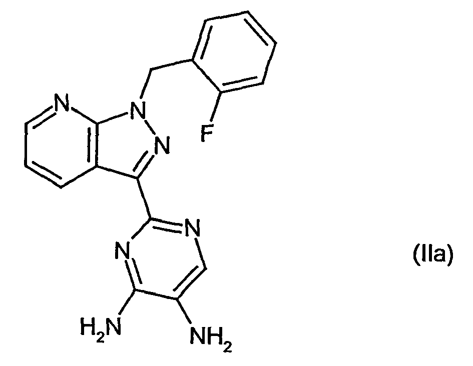 kann hergestellt werden durch Umsetzung der Verbindung der Formel (III) 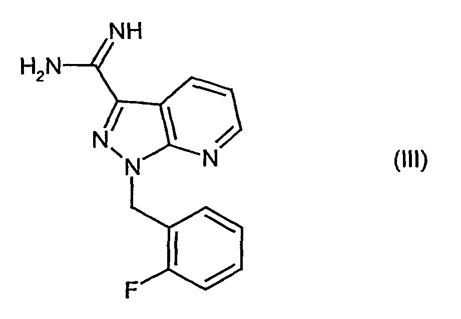 mit der Verbindung der Formel (IV) 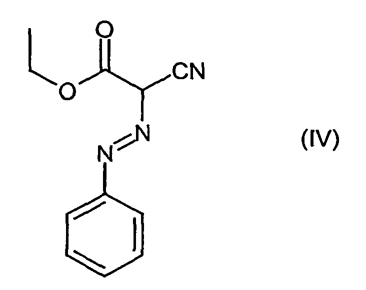 in einem organischen Lösungsmittel unter Erhitzen zu einer Verbindung der Formel (V)

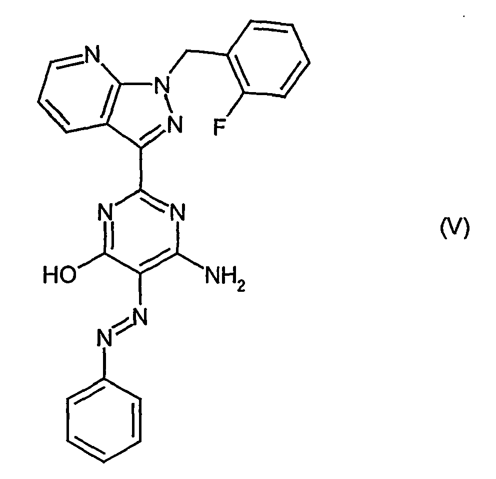

Umsetzung mit einem Reduktionsmittel wie Raney-Nickel in Gegenwart von Wasserstoff in einem organischen Lösungsmittel unter Erhitzen zu einer Verbindung der Formel (VI) 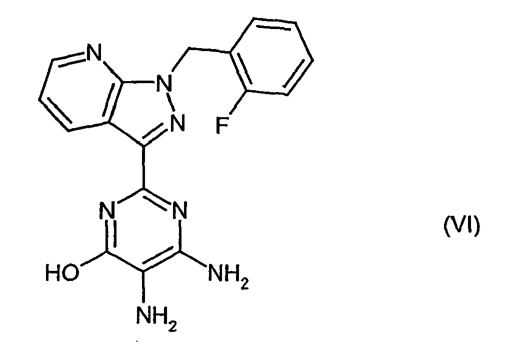 und Entfernung der Hydroxygruppe durch Umsetzung mit einem Chlorierungsmittel wie POCl₃ in Gegenwart einer organischen Base unter Erhitzen und anschließende Umsetzung mit Ammoniumformiat in Gegenwart eines Katalysators in einem organischen Lösungsmittel.

Die Ausgangsverbindung der Formel (IIb) 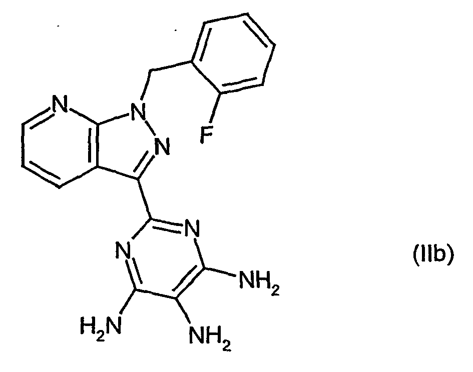 kann hergestellt werden durch Umsetzung der Verbindung der Formel (III) 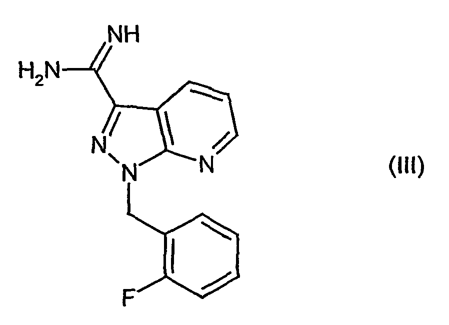 mit der Verbindung der Formel (VII) 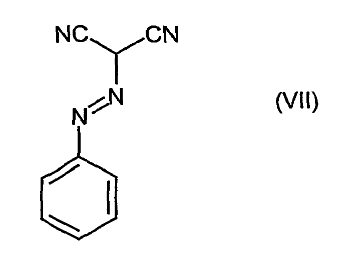 in einem organischen Lösungsmittel in Gegenwart einer Base unter Erhitzen zu einer Verbindung der Formel (VIII) 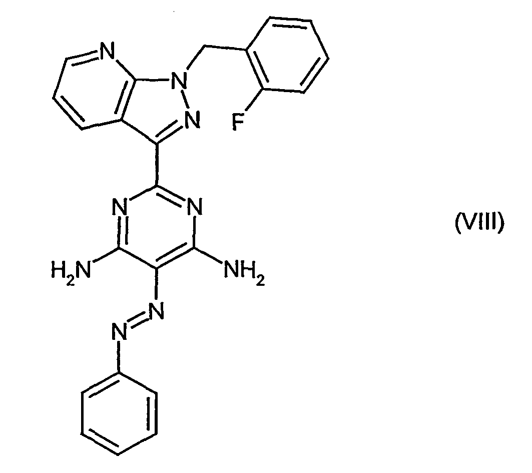 und Umsetzung mit einem Reduktionsmittel wie Raney-Nickel in einem organischen Lösungsmittel unter Erhitzen.

Die Verbindung der Formel (III) lässt sich gemäß folgendem Reaktionsschema herstellen:

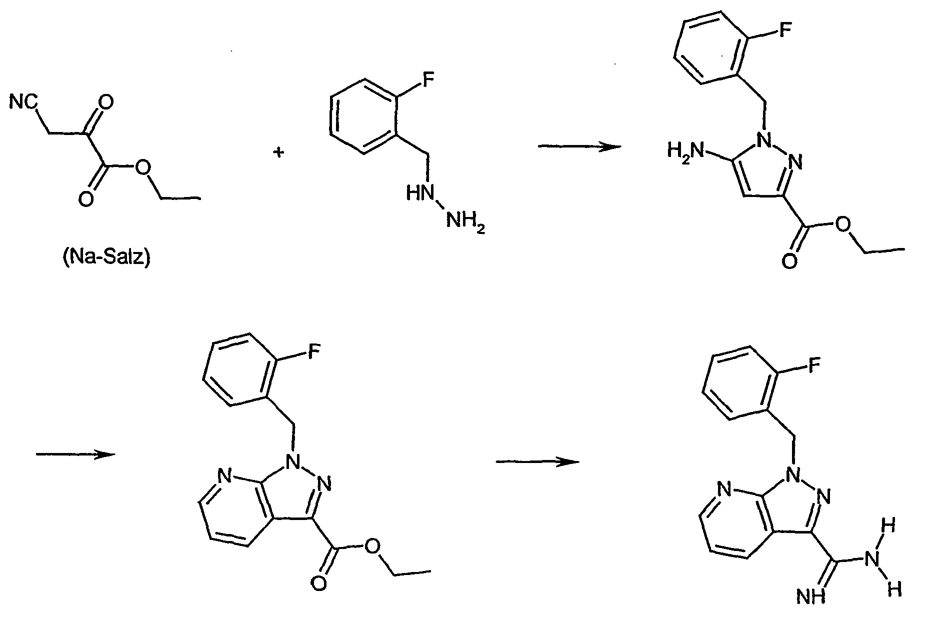

Die Verbindung der Formel (III) ist in einer mehrstufigen Synthese aus dem literaturbekannten Natriumsalz des Cyanobrenztraubensäureethylesters (Borsche und Manteuffel, Liebigs. Ann. Chem. 1934, 512, 97) erhältlich. Durch dessen Umsetzung mit 2-Fluorbenzylhydrazin unter Erhitzen und Schutzgasatmosphäre in einem inerten Lösungsmittel wie Dioxan erhält man den 5-Amino-1-(2-fluorbenzyl)-pyrazol-3-carbonsäureethylester, der durch Umsetzung mit Dimethylaminoacrolein im sauren Medium unter Schutzgasatmosphäre und Erhitzen zum entsprechenden Pyridinderivat cyclisiert. Dieses Pyridinderivat 1-(2-Fluorbenzyl)-1H-pyrazolo[3,4-b]pyridin-3-carbonsäureethylester wird durch eine mehrstufige Sequenz, bestehend aus Überführung des Esters mit Ammoniak in das entsprechende Amid, Dehydratisierung mit einem wasserentziehenden Mittel wie Trifluoressigsäureanhydrid zum entsprechenden Nitrilderivat, Umsetzung des Nitrilderivats mit Natriumethylat und abschließende Reaktion mit Ammoniumchlorid in die Verbindung der Formel (III) überführt.

Die Verbindung der Formel (IV) ist erhältlich aus Cyanessigsäureethylester und Anilin gemäß der Vorschrift von Menon R., Purushothaman E, J. Indian Chem. Soc. 74 (1997), 123.

Die Umsetzung der Verbindung der Formel (III) mit der Verbindung der Formel (IV) zur Verbindung der Formel (V) kann durch Umsetzung dieser Reaktanden vorzugsweise in äquimolaren Mengen gegebenenfalls in einem organischen Lösungsmittel, beispielsweise einem aromatischen Kohlenwasserstoff, insbesondere Toluol, vorzugsweise bei Normaldruck und Rühren der Reaktionslösung für mehrere Stunden, beispielsweise 12 Stunden, bei erhöhter Temperatur, beispielsweise 60-130°C, vorzugsweise unter Rückfluss des Lösungsmittels erfolgen.

Die Umsetzung der Verbindung der Formel (V) zur Verbindung der Formel (VI) kann durch Umsetzung mit Wasserstoff in Gegenwart eines für derartige Reaktionen herkömmlich eingesetzten Katalysators wie beispielsweise Raney-Nickel in einem für derartige Reaktionen herkömmlich eingesetzten organischen Lösungsmittel wie beispielsweise Dimethylformamid (DMF) (vgl. auch die Angaben zur Synthese der Verbindung VIII) vorzugsweise durch Anlegen von 30 bis 80 bar Wasserstoff, insbesondere 50 bis 70 bar Wasserstoff und Rühren der Reaktionslösung für mehrere Stunden, beispielsweise 24 Stunden, bei erhöhter Temperatur, beispielsweise 50-100°C, vorzugsweise bei 50 bis 80°C erfolgen.

Die Entfernung der Hydroxygruppe aus der Verbindung der Formel (VI) unter Erhalt der Verbindung der Formel (IIa) kann erfindungsgemäß bevorzugt über eine zweistufige Reaktion erfolgen. Im ersten Schritt wird gemäß der vorliegenden Erfindung die Hydroxygruppe durch eine Halogengruppe, vorzugsweise einen Chlorrest ersetzt. Dies kann durch Umsetzung der Verbindung der Formel (VI) mit einer vorzugsweise äquimolaren Menge eines Halogenierungsmittels, insbesondere eines Chlorierungsmittels wie beispielsweise POCl₃ in Gegenwart katalytischer Mengen einer organischen Base, beispielsweise einem Amin, vorzugsweise N,N-Dimethylanilin, gegebenenfalls in einem für derartige Reaktionen herkömmlich eingesetzten organischen Lösungsmittel vorzugsweise bei Normaldruck und Rühren der Reaktionslösung für mehrere Stunden, beispielsweise 3 bis 6 Stunden, bei erhöhter Temperatur, beispielsweise 60-130°C, vorzugsweise unter Rückfluss der Reaktionslösung erfolgen. Im zweiten Schritt wird dann der Halogenrest wie der Chlorrest auf dem Fachmann bekannte herkömmliche Art, beispielsweise durch Umsetzung mit einem Überschuss, beispielsweise einem sieben- bis fünfzehnfachen Überschuss an Ammoniumformiat in Gegenwart eines für derartige Reaktionen herkömmlich eingesetzten Katalysators wie beispielsweise Pd/C in einem für derartige Reaktionen herkömmlich eingesetzten organischen Lösungsmittel wie beispielsweise einem Alkohol, vorzugsweise Methanol, und Rühren der Reaktionslösung für mehrere Stunden bis zu mehreren Tagen, beispielsweise 1 bis 3 Tagen, bei erhöhter Temperatur, beispielsweise 50-130°C, vorzugsweise unter Rückfluss der Reaktionslösung entfernt.

Die Synthese der Verbindung der Formel (VII), Phenylazomalondinitril, aus Anilin und Malondinitril durch Diazotierung ist literaturbekannt (L.F.Cavalieri, J.F.Tanker, A.Bendich J.Am.Chem.Soc. 1949, 71,533).

Die Umsetzung der Verbindung der Formel (IIb) mit Phenylazomalondinitril (Verbindung der Formel (VII) erfolgt in Gegenwart einer Base. Als Basen können hierbei im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)-amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium und deren Hydride wie Natriumhydrid einzusetzen. Bevorzugt ist Natriummethanolat.

Als Lösemittel eignen sich hierbei inerte organische Lösemittel. Hierzu gehören Ether, wie Diethylether oder Tetrahydrofuran, DME, Dioxan, Alkohole wie Methanol und Ethanol, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Tetrahydrofuran, Dimethylformamid, Toluol, Dioxan oder Dimethoxyethan.

Die Reaktion wird unter Erhitzen auf Temperaturen zwischen 60°C und 110°C und Normaldruck durchgeführt. Man lässt das Reaktionsgemisch etwa 5-24 Stunden, vorzugsweise 12 bis 24 Stunden reagieren.

Anschließend wird durch Spaltung der Azogruppe die Verbindung der Formel (IIb) erhalten. Als Reduktionsmittel können hierfür Metalle, insbesondere Zink, in Gegenwart von Mineralsäuren wie Salzsäure, Na₂SO₄, Borane oder Wasserstoff in Gegenwart eines Katalysators verwendet werden. Erfindungsgemäß bevorzugt ist die Verwendung von Wasserstoff in Gegenwart von Raney-Nickel.

Als Lösemittel können die vorstehend genannten Lösemittel eingesetzt werden. Besonders bevorzugt ist hierbei Dimethylformamid (DMF) Die Reaktion wird vorzugsweise unter Erwärmen, beispielsweise bei 50-80°C, und einem Wasserstoffdruck von 30 bis 80 Bar, vorzugsweise 50 bis 70 Bar durchgeführt. Man lässt die Reaktionspartner etwa 24 Stunden reagieren.

Die so erhaltenen Verbindungen der Formel (II) können durch Umsetzung mit einer äquimolaren Menge oder vorzugsweise eines Überschusses, beispielsweise eines einbis fünffachen Überschusses, insbesondere eines ein- bis dreifachen Überschusses einer Sulfonylverbindung der Formel X-L-SO₂X, wobei X für eine durch eine Aminogruppe substituierbare Abgangsgruppe wie beispielsweise Halogen steht und L für eine Alkandiylgruppe oder eine Alkendiylgruppe mit jeweils 3 bis 5 Kohlenstoffatomen steht, wobei ein oder mehrere Kohlenstoffatome durch ein oder mehrere Heteroatome aus der Gruppe N, O, S ersetzt sein können, und wobei die Gruppe gegebenenfalls substituiert sein kann, in die erfindungsgemäßen Verbindungen der Formel (I) überführt werden. Die Reaktion erfolgt in zwei Schritten. Zunächst werden die Verbindungen der Formel (II) mit einer äquimolaren Menge oder vorzugsweise eines Überschusses, beispielsweise eines ein- bis fünffachen Überschusses, insbesondere eines ein- bis dreifachen Überschusses der vorstehend beschriebenen Sulfonylverbindungen der Formel X-L-SO₂X in Gegenwart einer Base wie einem organischen Amin, vorzugsweise Pyridin, vorzugsweise bei Normaldruck und Rühren der Reaktionslösung für mehrere Stunden, beispielsweise 12 Stunden, bei Raumtemperatur umgesetzt. Die vorstehend beschriebenen und hierbei verwendeten Sulfonylverbindungen sind käuflich erhältlich oder auf dem Fachmann bekannte Weise zugänglich. Anschließend werden die so erhaltenen Zwischenprodukte in einem organischen Lösungsmittel gelöst, mit einem Überschuss, beispielsweise einem ein- bis zehnfachen Überschuss, insbesondere einem drei- bis achtfachen Überschuss einer Base versetzt und vorzugsweise bei Normaldruck und Rühren der Reaktionslösung für mehrere Stunden, beispielsweise 12 Stunden, bei erhöhter Temperatur, beispielsweise 60-130°C, vorzugsweise, vorzugsweise 70-90°C zu den erfmdungsgemäßen Verbindungen der Formel (I) umgesetzt. Als Lösemittel eignen sich hierbei inerte organische Lösemittel. Hierzu gehören Ether, wie Diethylether oder Tetrahydrofuran, DME, Dioxan, Alkohole wie Methanol und Ethanol, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol,Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt Dimethylformamid. Als Basen können hierbei im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)-amine) wie Triethylamin, oder Heterocyclen wie 1,4-Diazabicyclo-[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium und deren Hydride wie Natriumhydrid einzusetzen. Bevorzugt ist Kaliumcarbonat.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I) führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen der allgemeinen Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektile Dysfunktion, weibliche sexuelle Dysfunktion, Osteoporose, Gastroparese und Inkontinenz eingesetzt werden.

Die in der vorliegenden Erfindung beschriebenen Verbindungen der allgemeinen Formel (I) stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Darüber hinaus umfasst die Erfindung die Kombination der erfindungsgemäßen Verbindungen der allgemeinen Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfasst die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindung potenziert und der gewünschte pharmakologische Effekt gesteigert.

### Biologische Untersuchungen

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10. Die Kontraktionskraft wird mit Statham UC2-Zellen erfaßt, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀). Das Standardapplikationsvolumen beträgt 5 µl, der DMSO-Anteil in der Badlösung entspricht 0,1 %. Die Ergebnisse sind nachstehend in Tabelle 1 aufgeführt:

**Tabelle 1: Gefäßrelaxierende Wirkung in vitro**

| Beispiel Nr. | IC₅₀[nM] |
|---|---|
| 1 | 290 |
| 3 | 350 |

### Bestimmung der Leberclearance in vitro

Ratten werden anästhesiert, heparinisiert, und die Leber in situ über die Pfortader perfundiert. Ex vivo werden dann aus der Leber mittels Collagenase-Lösung die primären Ratten-Hepatozyten gewonnen. Es wurden 2˙10⁶ Hepatozyten pro ml mit jeweils der gleichen Konzentration der zu untersuchenden Verbindung bei 37°C inkubiert. Die Abnahme des zu untersuchenden Substrates über die Zeit wurde bioanalytisch (HPLC/UV, HPLC/Fluoreszenz oder LC/MSMS) an jeweils 5 Zeitpunkten im Zeitraum von 0-15 min nach Inkubationsstart bestimmt. Daraus wurde über Zellzahl und Lebergewicht die Clearance errechnet.

### Bestimmung der Plasmaclearance in vivo

Die zu untersuchende Substanz wird Ratten über die Schwanzvene intravenös als Lösung appliziert. Zu festgelegten Zeitpunkten wird den Ratten Blut entnommen, dieses wird heparinisiert und durch herkömmliche Maßnahmen Plasma daraus gewonnen. Die Substanz wird im Plasma bioanalytisch quantifiziert. Aus den so ermittelten Plasmakonzentrations-Zeit-Verläufen werden über herkömmliche hierfür verwendete nicht-kompartimentelle Methoden die pharmakokinetischen Parameter errechnet.

Zur vorliegenden Erfindung gehören pharmazeutische Zubereitungen, die neben nichttoxischen, inerten pharmazeutisch geeigneten Trägerstoffen die erfindungsgemäßen Verbindungen der allgemeinen Formel (I) enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen der allgemeinen Formel (I) sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen der allgemeinen Formel (I) auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 0,1 bis etwa 80, insbesondere 0,1 bis 30mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- MCPBA:: m-Chlorperoxybenzoesäure
- BABA:: n-Butylacetat/n-Butanol/Eisessig/Phosphatpuffer pH 6 (50:9:25.15; org. Phase)
- DMF:: N,N-Dimethylformamid

### Laufmittel für die Dünnschichtchromatographie:

- T1 E1:: Toluol - Essigsäureethylester (1:1)
- T1 EtOH1:: Toluol-Methanol (1:1)
- C1 E1:: Cyclohexan - Essigsäureethylester (1:1)
- C1 E2:: Cyclohexan - Essigsäureethylester (1:2)

### Methoden zur Ermittlung der HPLC-Retentionszeiten:

### Methode A (HPLC-MS):

Eluent: A= CH₃CN B=0.6 g 30%ige HCl /l H₂O
Fluss: 0.6 ml/min
Säulenofen: 50°C
Säule: Symmetry C18 2.1*150mm
Gradient:

| Zeit (min) | %A | %B | Fluss (ml/min) |
|---|---|---|---|
| 0 | 10 | 90 | 0.6 |
| 4 | 90 | 10 | 0.6 |
| 9 | 90 | 10 | 0.8 |

### Methode B (HPLC):

Eluent: A=5 ml HClO₄/l H₂O, B=CH₃CN
Fluss: 0.75 ml/min
L-R Temperatur: 30.00°C 29.99°C
Säule: Kromasil C18 60*2mm
Gradient:

| Zeit (min) | %A | %B |
|---|---|---|
| 0.50 | 98 | 2 |
| 4.50 | 10 | 90 |
| 6.50 | 10 | 90 |
| 6.70 | 98 | 2 |
| 7.50 | 98 | 2 |

### Methode C (HPLC):

Eluent: A= H₃PO₄ 0.01 mol/l, B=CH₃CN
Fluss: 0.75 ml/min
L-R Temperatur: 30.01°C 29.98°C
Säule: Kromasil C18 60*2mm
Gradient:

| Zeit (min) | %A | %B |
|---|---|---|
| 0.00 | 90 | 10 |
| 0.50 | 90 | 10 |
| 4.50 | 10 | 90 |
| 8.00 | 10 | 90 |
| 8.50 | 90 | 10 |
| 10.00 | 90 | 10 |

### Methode D (chirale HPLC):

- Eluent:: 50% iso-Hexan, 50% Ethanol
- Fluss:: 1.00 ml/min
- Temperatur:: 40°C
- Säule:: 250*4,6 mm, gefüllt mit Chiralcel OD, 10 µm

### Methode E (HPLC-MS):

Eluent: A= CH₃CN B=0.3 g 30%ige HCl /l H₂O
Fluss: 0.9 ml/min
Säulenofen: 50°C
Säule: Symmetry C18 2.1*150mm
Gradient:

| Zeit (min) | %A | %B | Fluss (ml/min) |
|---|---|---|---|
| 0 | 10 | 90 | 0.9 |
| 3 | 90 | 10 | 1.2 |
| 6 | 90 | 10 | 1.2 |

### Ausgangsverbindungen:

### I. Synthese von 1-(2-Fluorbenzyl)1H-pyrazolo[3,4-b]pyridin-3-carboxamidin

### 1A) 5-Amino-1-(2-fluorbenzyl)-pyrazol-3-carbonsäureethylester

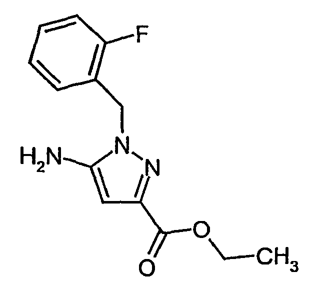

100 g (0.613 mol) Natriumsalz des Cyanobrenztraubensäureethylester (Darstellung analog Borsche und Manteuffel, Liebigs Ann. 1934, 512, 97) werden unter gutem Rühren unter Argon in 2.5 1 Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### 1B) 1-(2-Fluorbenzyl)-1H-pyrazolo[3,4-b]pyridin-3-carbonsäureethylester

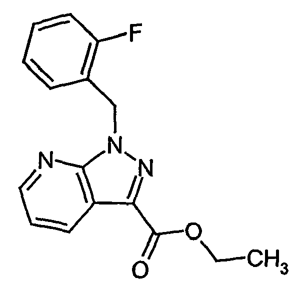

Die aus 1A) erhaltene Lösung wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum verdampft, der Rückstand in 2 1 Wasser gegeben und dreimal mit je 1 1 Essigester extrahiert. Die vereinigten organischen Phasen werden mit Magnesiumsulfat getrocknet und einrotiert. Man chromatographiert auf 2.5 kg Kieselgel und eluiert mit einem Toluol / Toluol-Essigeste=4:1 -Gradienten. Ausbeute: 91.6 g (49.9 % d.Th. über zwei Stufen).
Smp. 85 °C
R_{f} (SiO₂, T1E1): 0.83

### 1C) 1-(2-Fluorbenzyl)-1H-pyrazolo[3,4-b]pyridin-3-carboxamid

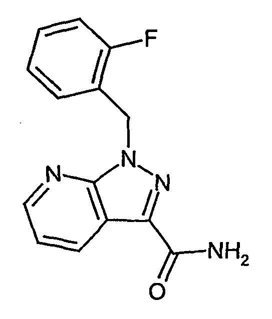

10.18 g (34 mmol) des in Beispiel 1B) erhaltenen Esters werden in 150 ml mit Ammoniak bei 0 - 10°C gesättigtem Methanol vorgelegt. Man rührt zwei Tage bei Raumtemperatur und engt anschließend im Vakuum ein.
R_{f} (SiO₂, T1E1): 0.33

### 1D) 3-Cyano-1-(2-fluorbenzyl)-1H-pyrazolo[3,4-b]pyridin

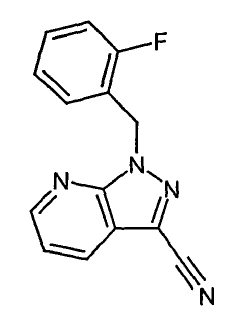

36.1 g (133 mmol) 1-(2-Fluorbenzyl)-1H-pynzolo[3,4-b]pyridin-3-carboxamid aus Beispiel 1C) werden in 330 ml THF gelöst und mit 27 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40 °C ansteigt. Man rührt über Nacht bei Raumtemperatur. Anschließend wird der Ansatz in 1l Wasser gegeben und dreimal mit je 0.5 l Essigester extrahiert. Die organische Phase wird mit gesättigter Natriumhydrogencarbonatlösung und mit 1 N HCl gewaschen, mit MgSO4 getrocknet und einrotiert.
Ausbeute: 33.7 g (100% d.Th.)
Smp: 81°C
R_{f} (SiO₂, TIE1): 0.74

### 1E) (2-Fluorbenzyl)-1H-pyrazolo[3,4-b]pyridin-3-carboximidsäuremethylester

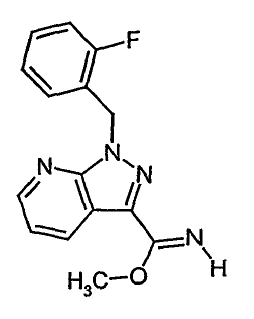

Man löst 30.37 g (562 mmol) Natriummethylat in 1.5 l Methanol und gibt 36.45 g (144.5 mmol) 3-Cyano-1-(2-fluorbenzyl)-1H-pyrazolo[3,4-b]pyridin (aus Beispiel 1D) hinzu. Man rührt 2 Stunden bei Raumtemperatur und setzt die erhaltene Lösung direkt für die nächste Stufe ein.

### 2F) 1-(2-Fluorbenzyl)1H-pyrazolo[3,4-b]pyridin-3-carboxamidin

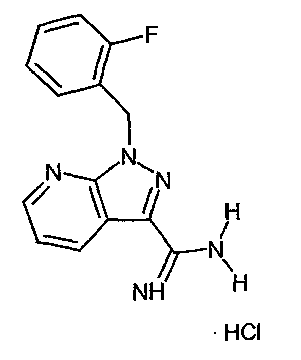

Die aus Beispiel 1E) erhaltene Lösung von (2-Fluorbenzyl)-1H-pyrazolo[3,4-b]pyridin-3-carboximidsäuremethylester in Methanol wird mit 33.76 g (32.19 ml, 562 mmol) Eisessig und 9.28 g (173 mmol) Ammoniumchlorid versetzt und über Nacht unter Rückfluss gerührt. Man verdampft das Lösungsmittel im Vakuum, verreibt den Rückstand gut mit Aceton und saugt den ausgefallenen Feststoff ab.
¹H-NMR (d₆-DMSO, 200 MHz): δ= 5,93 (s, 2H); 7,1-7,5 (m, 4 H); 7,55 (dd, 1H); 8,12 (dd, 1H); 8,30 (dd, 1H); 9,5 (bs, 4H-austauschbar) ppm.
MS (EI): m/z = 270,2 (M-HCl)

### II. Synthese von 6-Amino-2-[1-(2-fluorobenzyl)-1H-pyrazolo[3,4-b]pyridin-3-yl]-5-[(E)-phenyldiazenyl]-4-pyrimidinol

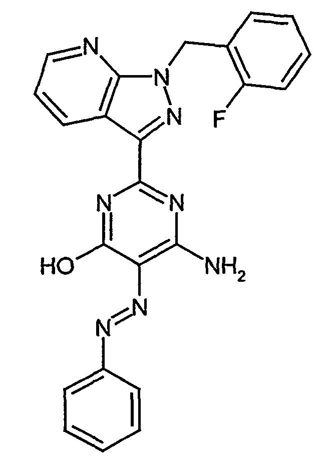

2.43 g (9.02 mmol) 1-(2-Fluorobenzyl)-1H-pyrazolo[3,4-b]pyridin-3-carboximidamid aus Bsp. I und Ethylcyano[(*E*)-phenyldiazenyl]acetat (1.96 g, 9.02 mmol) wurden für 12 h unter Rückfluss erhitzt. Nach Abkühlen auf Raumtemperatur filtrierte man vom ausgefallenen Niederschlag und wusch diesen mehrfach mit Toluol. Flash-Chromatographie (CH₂Cl₂/Ethylacetat 50:1 -> EE) lieferte das gewünschte Produkt.
- Ausbeute:: 2.52 g (63%)
- R_{f}-Wert:: 0.72 (CH₂Cl₂/MeOH 20/1)
- ¹H-NMR:: (300 MHz, D₆-DMSO), δ = 5.87 (s, 2 H, 2 x CH₂O)7.17 (t, 1 H, Ar-H), 7.25 (d, 1 H, Ar-H), 7.3-7.6 (m, 6 H, Ar-H" NH₂), 7.80 (d, 2 H, Ar-H), 8.75 (br. s, 2 H, Ar-H), 9.05 (d, 1 H), 10.23 (br. s, 1 H), 12.1 (br. s, 1 H).
- LCMS:: Ret.-zeit: 3.94 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90)); MS: (ESI pos.), *m*/*z* = 441 ([M+H]⁺), (ESI neg.), *m*/*z* = 439 ([M-H]⁻)

### III. Synthese von 5,6-Diamino-2-[1-(2-fluorobenzyl)-1H-pyrazolo[3,4-b]pyridin-3-yl]-4-pyrimidinol

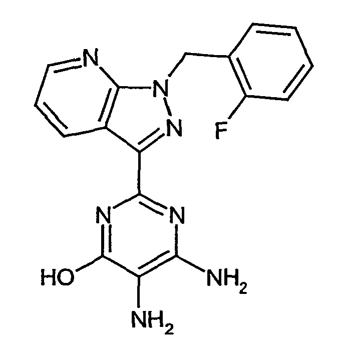

6-Amino-2-[1-(2-fluorobenzyl)-1H-pyrazolo[3,4-b]pyridin-3-yl]-5-[(*E*)-phenyldiazenyl]-4-pyrimidinol (2.52 g, 5.72 mmol) aus Bsp. II und Raney-Ni (50% in H₂O, 0.217 g) wurden in DMF gelöst und bei 65 bar H₂ für 22 h bei 62°C hydriert. Nach Abkühlen nahm man in DMF auf, erhitzte auf 100°C und filtrierte vom Katalysator. Die Mutterlauge wurde mit 10 ml HCl (5 N) und H₂O (20 ml) versetzt. Nach 30 min Rühren bei Raumtemperatur filtrierte man vom ausgefallenen Niederschlag und wusch mit H₂O nach.
- Ausbeute:: 1.94 g (97 %)
- R_{f}-Wert:: 0.10 (CH₂Cl₂/MeOH 10:1)
- ¹H-NMR:: (300 MHz, D₆-DMSO), δ = 5.78 (s, 2H, OCH₂), 5.90 (s, 2 H, NH₂), 7.1-7.4 (m, 7 H, Ar-H, NH₂), 8.64 (d, 1 H, Ar-H), 8.85 (s, 1 H, Ar-H), 11.7 (br. s, 1 H, OH).
- LC-MS:: Retentionszeit: 2.74 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90)); MS: (ESI pos.), *m*/*z* = 352 ([M+H]⁺), (ESI neg.), *m*/*z* = 350 ([M-H]⁻)

### IV. Synthese von 6-Chloro-2-[1-(2-fluorobenzyl)-1H-pyrazolo[3,4-b]pyridin-3-yl]-4,5-pyrimidindiamin

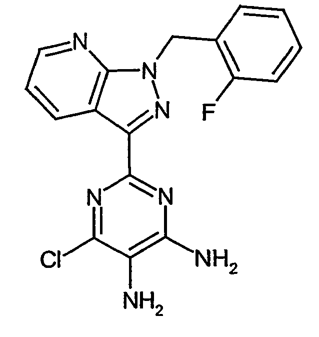

5,6-Diamino-2-[1-(2-fluorobenzyl)-1H-pyrazolo[3,4-b]pyridin-3-yl]-4-pyrimidinol (0.15 mg, 0.42 mmol) aus Bsp. III wurde in POCl₃ (5 ml) gelöst und mit N,N-Dimethylanilin (5.0 mg, 0.04 mmol, 0.1 Äquivalente) versetzt. Man erhitzte für 4 h unter Rückfluss und entfernte dann das überschüssige Reagenz im Vakuum. Man nahm in Essigsäureethylester auf und wusch mit ges. wässriger NaHCO₃-Lösung, H₂O und gesättigter wässriger NaCl-Lösung. Die vereinigten organischen Phasen wurden eingeengt und per Flash-Chromatographie gereinigt (CH₂Cl₂:MeOH 40:1).
- Ausbeute:: 0.12 g (77 %)
- R_{f}-Wert:: 0.60 (CH₂Cl₂/MeOH 10:1)
- LCMS:: Retentionszeit: 3.70 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90)); MS: (ESI pos.), *m*/*z* = 370 ([M+H]⁺), (ESI neg.), *m*/*z* = 368 ([M-H]⁻)

### V. Synthese von 2-[1-(2-Fluorobenzyl)-1H-pyrazolo[3,4-b]pyridin-3-yl]-4,5-pyrimidindiamin

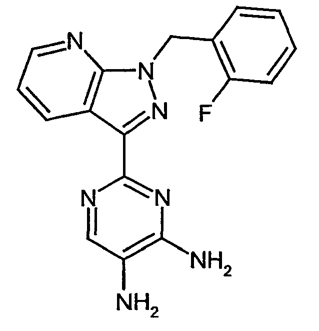

6-Chloro-2-[1-(2-fluorobenzyl)-1H-pyrazolo[3,4-b]pyridin-3-yl]-4,5-pyrimidindiamin (74 mg, 0.20 mmol) aus Bsp. V wurde in MeOH (4 ml) gelöst und mit Pd/C (10%, 20 mg) und Ammoniumformiat (126 mg, 2.00 mmol, 10 Äquivalente) versetzt. Man erhitzte für 2 Tage unter Rückfluss und ließ dann auf Raumtemperatur abkühlen, bevor vom Katalysator filtriert wurde. Die Reinigung erfolgte per präparativer HPLC (Säule: Cromsil 120 ODS, C-18, 10 µm, 250X30 mm, Fluss 50 ml/min, Raumtemperatur, Gradient: Wasser Acetonitril bei 0 min: 90:10, bei 28 min 5:95) lieferte das gewünschte Produkt.
- Ausbeute:: 0.054 g (80 %)
- R_{f}-Wert:: 0.10 (CH₂Cl₂/MeOH 20:1)
- ¹H-NMR:: (300 MHz, D₆-DMSO), δ = 5.90 (s, 2H, OCH₂), 7.1-7.6 (m, 6 H, Ar-H), 8.75 (d, 1 H, Ar-H), 8.98 (d, 1 H, Ar-H).
- LCMS:: Ret.-zeit: 2.66 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90)); MS: (ESI pos.), *m*/*z* = 336 ([M+H]⁺), (ESI neg.), *m*/*z* = 334 ([M-H]⁻)

### VI. Synthese von 2-[1-(2-Fluorobenzyl)-1H-pyrazolo[3,4-b]pyridin-3-yl]-5-[(E)-phenyldiazenyl]-4,6-pyrimidindiamin

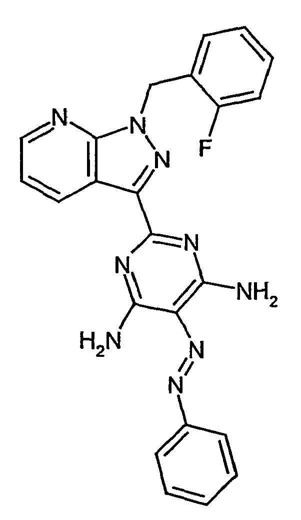

Man gibt zu einer gerührten Lösung von 21.92 g (71.7 mmol) 1-(2-Fluorbenzyl)1H-pyrazolo[3,4-b]pyridin-3-carboxamidin in Toluol aus Beispiel I 3.87 g Natriummethanolat und anschließend 12.2 g (71.7 mmol) Phenylazomalononitril (L.F.Cavalieri, J.F.Tanker, A.Bendich J.Am.Chem.Soc., 1949, 71, 533). Man rührt über Nacht bei 110°C und lässt abkühlen. Der hierbei ausgefallene Feststoff wird abgesaugt und mit Ethanol gewaschen. Nach Trocknung erhält man 23 g (73 % d.Th.) der gewünschten Verbindung.
- R_{f}-Wert:: 0.50 (Toluol/EE 1:1)
- ¹H-NMR:: (300 MHz, D₆-DMSO), δ = 5.88 (s, 2H, OCH₂), 7.1-7.5 (m, 7 H, Ar-H), 7.87 (br. s, 2 H, NH₂), 7.96 (s, 2 H, Ar-H), 8.00 (s, 1 H, Ar-H), 8.03 (s, 1 H, Ar-H), 8.48 (br. s, 2 H, NH₂), 8.65 (d, 1 H, Ar-H), 9.20 (d, 1 H, Ar-H)
- MS:: (ESI pos.), *m*/*z* = 440 ([M+H]⁺)

### VII. Synthese von 2-[1-(2-Fluorobenzyl)-1H-pyrazolo[3,4-b]pyridin-3-yl]-4,5,6-pyrimidintriamin Trihydrochlorid

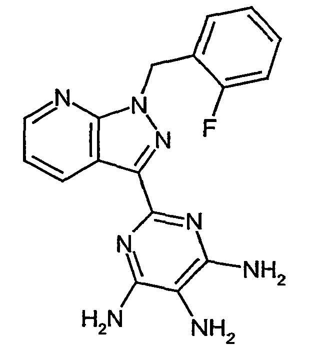

5 g (11.38 mmol) 2-[1-(2-Fluorobenzyl)-1H-pyrazolo[3,4-b]pyridin-3-yl]-5-[(E)-phenyldiazenyl]-4,6-pyrimidindiamin aus Beispiel VI werden mit 800 mg 50proz. Raney-Nickel in Wasser in 60 ml DMF 22 Stunden lang bei 65 bar Wasserstoffdruck und 62°C hydriert. Man saugt vom Katalysator über Kieselgur ab, dampft die Lösung im Vakuum ein und rührt mit 5 N HCl. Der ausgefallene gelbbraune Niederschlag wird abgesaugt und getrocknet. Man erhält 3.1 g (59.3 % d. Th.) der gewünschten Verbindung. Die freie Base erhält man durch Ausschütteln mit verdünnter NaHCO₃-Lösung und extrahieren mit Essigsäureethylester. Der in beiden Phasen unlösliche Feststoff wird abgesaugt. Auch die Essigsäureethylesterphase enthält geringe Mengen der freien Base.
- R_{f}-Wert:: 0.18 (EE)
- ¹H-NMR:: (300 MHz, D₆-DMSO), δ = 4.45 (br. s, 6 H, NH₂), 5.92 (s, 2H, OCH₂), 7.1-7.6 (m, 5 H, Ar-H), 8.76 (d, 2 H, Ar-H), 8.98 (d, 1 H, Ar-H)

### VIII. 6-Amino-5-(1,1-dioxido-2-isothiazolidinyl)-2-[1-(2-fluorobenzyl)-1H-pyrazolo[3,4-b] pyridin3-yl]-4-pyrimidinol

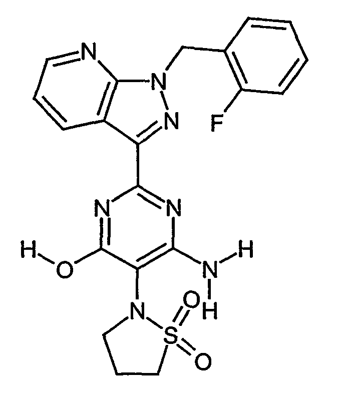

5,6-Diamino-2-[1-(2-fluorobenzyl)-1H-pyrazolo[3,4-b]pyridin-3-yl]-4-pyrimidinol (0.25 g, 0.71 mmol) aus Bsp. III wurde in Pyridin (2.5 ml) vorgelegt und mit 3-Chlorpropansulfonylchlorid (0.19 g, 1.1 mmol, 1.5 Äquiv.) versetzt. Nach 12 h bei Raumtemperatur engte man im Vakuum ein und löste in DMF (2.5 ml). Man versetzte mit K₂CO₃ (0.69 g, 5.0 mmol, 7 Äquiv.) und rührte für 20 h bei 40°C nach. Man nahm in Essigsäureethylester und H₂O auf und extrahierte mehrfach mit Essigsäureethylester. Die vereinigten organischen Phasen wurden über Na₂SO₄ getrocknet und im Vakuum eingeengt. Die so erhaltenen Kristalle wurden mit CH₃CN verrührt, abgesaugt und im Vakuum getrocknet.
- Ausbeute:: 0.128 g (39 %)
- ¹H-NMR:: (300 MHz, D₆-DMSO), δ = 2.43 (br. s, 2 H, CH₂), 3.29 (br. s, 2 H, CH₂), 3.60 (br. d, 2 H, CH₂), 5.82 (s, CH₂O), 6.8 (br. s, 2 H, NH₂), 7.1-7.5 (m, 5 H, Ar-H), 8.45 (s, 1 H, Ar-H), 8.69 (d, 1 H, Ar-H), 8.90 (d, 1 H, Ar-H), 11.93 (s, 1 H, OH).
- LCMS:: Retentionszeit: 3.36 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90));
MS: (ESI pos.), *m*/*z* = 456 ([M+H]⁺), (ESI neg.), *m*/*z* = 4.54 ([M-H]⁻)

### Beispiele

### 1. 6-Chloro-5-(1,1-dioxido-2-isothiazolidinyl)-2-[1-(2-fluorobenzyl)-1H-pyrazolo[3,4-b]pyridin-3-yl]-4-pyrimidinamin

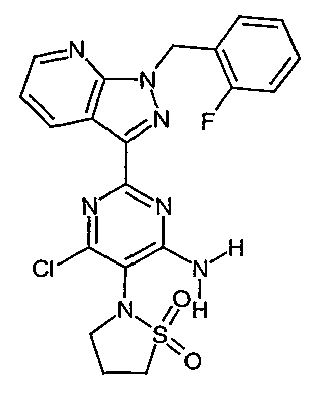

6-Amino-5-(1,1-dioxido-2-isothiazolidinyl)-2-[1-(2-fluorobenzyl)-1H-pyrazolo[3,4-b]pyridin3-yl]-4-pyrimidinol (0.080 g, 0.18 mmol) aus Bsp. VIII und Dichlorphenylphosphinoxid (2.0 ml) wurden bei 160°C für 2 h gerührt. Nach Abkühlen hydrolysierte man mit Eiswasser und reinigte das Rohprodukt per präparativer HPLC (Säule: Cromsil 120 ODS, C-18, 10 µm, 250X30 mm, Fluss 50 ml/min, Raumtemp., Gradient: Wasser Acetonitril bei 0 min: 90:10, bei 28 min 5:95).
- Ausbeute:: 0.050 g (60 %)
- ¹H-NMR:: (300 MHz, D₆-DMSO), δ = 2.55 (t, 2 H, CH₂), z. T. überlagert von DMSO, 3.36 (t, 2 H, CH₂), z. T. überlagert von H₂O, 3.64 (tt, 2 H, CH₂), 5.84 (s, CH₂O), 7.1-7.5 (m, 5 H, Ar-H), 7.9 (br. s, 2 H, NH₂), 8.66 (dd, 1 H, Ar-H), 8.91 (d, 1 H, Ar-H).
- LCMS:: Ret.-zeit: 3.90 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90)); MS: (ESI pos.), *m*/*z* = 474 ([M+H]⁺), (ESI neg.), *m*/*z* = 472 ([M-H]⁻)

### 2. 5-(1,1-dioxido-2-isothiazolidinyl)-2-[1-(2-fluorobenzyl)-1H-pyrazolo[3,4-b]pyridin-3-yl]-4-pyrimidinylamin

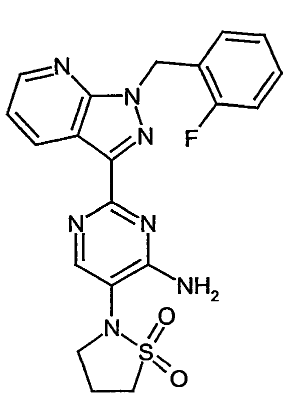

2-[1-(2-Fluorobenzyl)-1H-pyrazolo[3,4-b]pyridin-3-yl]-4,5-pyrimidindiamin (50 mg, 0.15 mmol) aus Bsp. V in Pyridin (1.3 ml) wurde bei Raumtemperatur mit 3-Chloropropansulfonylchlorid (79 mg, 0.45 mmol, 3 Äquivalente) versetzt. Man rührte für 12 h bei Raumtemperatur und versetzte mit weiterem 3-Chloropropansulfonylchlorid (39 mg, 0.23 mmol, 1.5 Äquivalente). Nach 6 h entfernte man überschüssiges Reagenz im Vakuum und reinigte per Flash-Chromatographie (CH₂Cl₂:MeOH 20:1). Das so erhaltene Zwischenprodukt wurde in DMF (1 ml) aufgenommen, mit K₂CO₃ (144 mg, 1.04 mmol, 7 Äquivalente) versetzt und für 12 h bei 80°C erhitzt. Anschließend entfernte man DMF im Vakuum und reinigte per Flash-Chromatographie (CH₂Cl₂:MeOH 20:1).
- Ausbeute:: 0.021 g (32 %)
- R_{f}-Wert:: 0.45 (CH₂Cl₂/MeOH 20:1)
- ¹H-NMR:: (300 MHz, D₆-DMSO), δ = 2.60 (tt, 2 H, CH₂), 3.41 (t, 2 H, CH₂), 3.75 (t, 2 H, CH₂), 5.70 (br. s, 2H, NH₂), 5.94 (s, CH₂O), 6.9-7.4 (m, 5 H, Ar-H, NH₂), 8.45 (s, 1 H, Ar-H), 8.60 (d, 1 H, Ar-H), 8.91 (d, 1 H, Ar-H).
- LCMS:: Ret.-zeit: 3.10 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90)); MS: (ESI pos.), *m*/*z* = 440 ([M+H]⁺), (ESI neg.), *m*/*z* = 438 ([M-H]⁻)

Alternativ wurde 5-(1,1-dioxido-2-isothiazolidinyl)-2-[1-(2-fluorobenzyl)-1H-pyrazolo[3,4-b]pyridin-3-yl]-4-pyrimidinylamin (Bsp. 2) hergestellt aus 6-Chloro-5-(1,1-dioxido-2-isothiazolidinyl)-2-[1-(2-fluorobenzyl)-1H-pyrazolo[3,4-b]pyridin-3-yl]-4-pyrimidinamin (0.040 mg, 0.084 mmol) aus Bsp. 1, das in MeOH (4 ml) gelöst und mit Pd/C (10%, 0.009 g) und Ammoniumformiat (0.053 mg, 0.84 mmol, 10 Äquiv.) versetzt wurde. Man erhitzte für 2 Tage unter Rückfluss und ließ dann auf Raumtemperatur abkühlen, bevor vom Katalysator filtriert wurde. Das Rohprodukt wurde im Vakuum eingeengt.
- Ausb.:: 0.007 g (19 %)

Die spektroskopischen Daten waren identisch mit dem über die vorstehend beschriebene Route hergestellten Produkt.

### 3. 5-(1,1-dioxido-2-isothiazolidinyl)-2-[1-(2-fluorobenzyl)-1H-pyrazolo[3,4-b]pyridin-3-yl]-4,6-pyrimidindiamin

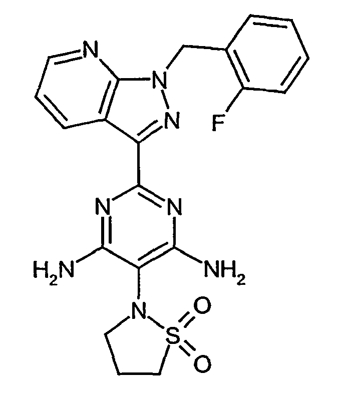

2-[1-(2-Fluorobenzyl)-1H-pyrazolo[3,4-b]pyridin-3-yl]-4,5,6-pyrimidintriamin (0.30 g, 0.86 mmol) aus Bsp. VII wurde bei Raumtemperatur in Pyridin (2 ml) gelöst und mit 3-Chlorpropansulfonylchlorid (0.23 g, 1.3 mmol, 1.5 Äquivalente) versetzt. Man rührte für 12 h bei Raumtemperatur und entfernte anschließend das Lösungsmittel am Rotationsverdampfer. Das so erhaltene Rohprodukt wurde in DMF (1 ml) gelöst und mit K₂CO₃ (0.83 g, 6.0 mmol, 7 Äquivalente) versetzt. Man rührte für 12 h bei 80°C. Die Rohmischung wurde per präparativer HPLC (Säule: Cromsil 120 ODS, C-18, 10 µm, 250X30 mm, Fluss 50 ml/min, Raumtemp., Gradient: Wasser Acetonitril bei 0 min: 90:10, bei 28 min 5:95) gereinigt.
- Ausbeute:: 0.22 g (55 %)
- R_{f}-Wert:: 0.25 (CH₂Cl₂/MeOH 20:1)
- ¹H-NMR:: (400 MHz, D₆-DMSO), δ = 2.45 (tt, 2 H, CH₂, z.T. überlagert von DMSO), 3.46 (t, 2 H, CH₂), z.T. überlagert von 3.50 (t, 2 H, CH₂), 5.81 (s, CH₂O), 6.6 (br. s, 4 H, 2 x NH₂), 7.1-7.4 (m, 5 H, Ar-H), 8.62 (m_{c}, 1 H, Ar-H), 9.03 (d, 1 H, Ar-H).
- LCMS:: Ret.-zeit: 2.80 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min; 90:10, bei 6.0 min 10:90)); MS: (ESI pos.), *m*/*z* = 455 ([M+H]⁺), (ESI neg.), *m*/*z* = 499 ([M-H, +HCOOH]⁻)

### 4. 5-(1,1-Dioxido-1,2-thiazinan-2-yl)-2-[1-(2-fluorobenzyl)-1H-pyrazolo[3,4-b]pyridin-3-yl]-4,6-pyrimidindiamin

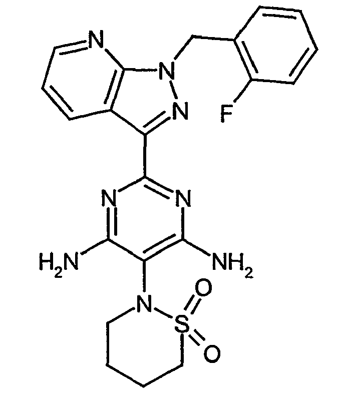

2-[1-(2-Fluorobenzyl)-1H-pyrazolo[3,4-b]pyridin-3-yl]-4,5,6-pyrimidintriamin (0.080 g, 0.23 mmol) aus Bsp. VII wurde bei Raumtemperatur in Pyridin (5 ml) gelöst und mit 4-Chlorbutansulfonylchlorid (0.065 g, 0.34 mmol, 1.5 Äquivalente; Darstellung aus Tetrahydrothiophen gemäß Runge et al., J. Prakt. Chem. 1955, 279, 288) versetzt. Man rührte für 12 h bei Raumtemperatur und entfernte anschließend das Lösungsmittel am Rotationsverdampfer. Das so erhaltene Rohprodukt wurde in DMF (1 ml) gelöst und mit K₂CO₃ (0.22 g, 1.6 mmol, 7 Äquivalente) versetzt. Man rührte für 12 h bei 80°C. Die Rohmischung wurde per präparativer HPLC (Säule: Cromsil 120 ODS, C-18, 10 µm, 250X30 mm, Fluss 50 ml/min, Raumtemp., Gradient: Wasser Acetonitril bei 0 min: 90:10, bei 28 min 5:95) gereinigt.
- Ausbeute:: 0.022 g (19%)
- R_{f}-Wert:: 0.25 (CH₂Cl₂/MeOH 20:1)
- ¹H-NMR:: (300 MHz, D₆-DMSO), δ = 1.90 (m_{c}, 2 H, CH₂), 2.15 (m_{c}, 2 H, CH₂), 3.48 (m_{c}, 4 H, 2 x CH₂), 5.80 (s, CH₂O), 6.48 (br. s, 4 H, 2 x NH₂), 7.1-7.4 (m, 5 H, Ar-H), 8.61 (m_{c}, 1 H, Ar-H), 9.05 (d, 1 H, Ar-H).
- LCMS:: Retentionszeit: 2.90 min (Säule: Symmetry, C-18, 3.5 µm, 50X2.1 mm, Fluss 0.5 ml/min, 40°C, Gradient: Wasser (+0.1% Ameisensäure):Acetonitril (+0.1% Ameisensäure) bei 0 min: 90:10, bei 6.0 min 10:90)); MS: (ESI pos.), *m*/*z* = 469 ([M+H]⁺)

## Patentansprüche

1. Verbindungen der allgemeinen Formel (I) 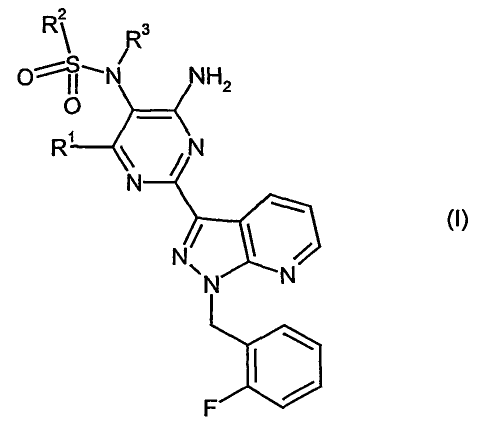 worin
R¹ für H, Cl oder NH₂ steht;
R² und R³ zusammen mit den Heteroatomen, an welche sie gebunden sind, einen fünf- bis siebengliedrigen Heterocyclus bilden, der gesättigt oder teilweise ungesättigt sein kann, gegebenenfalls ein oder mehrere weitere Heteroatome aus der Gruppe N, O, S enthalten kann und gegebenenfalls substituiert sein kann;
sowie Salze, Isomere und Hydrate davon.

2. Verbindungen nach Anspruch 1,
worin
R¹ für H, Cl oder NH₂ steht;
R² und R³ zusammen mit den Heteroatomen, an welche sie gebunden sind, einen fünf- bis siebengliedrigen Heterocyclus bilden, der gesättigt oder teilweise ungesättigt sein kann, gegebenenfalls ein oder mehrere weitere Heteroatome aus der Gruppe N, O, S enthalten kann;
sowie Salze, Isomere und Hydrate davon.

3. Verbindungen nach Anspruch 1,
worin
R¹ für H, Cl oder NH₂ steht;
R² und R³ zusammen mit den Heteroatomen, an welche sie gebunden sind, einen gesättigten fünf- bis siebengliedrigen Heterocyclus bilden;
sowie Salze, Isomere und Hydrate davon.

4. Verfahren zur Herstellung von Verbindungen der Formel 1, umfassend die Umsetzung der Verbindung der Formel (II) 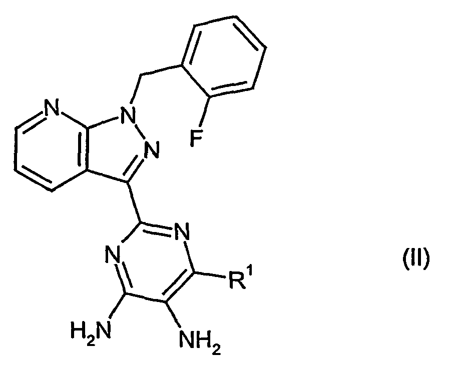 wobei R¹ wie vorstehend definiert ist;
mit einer Verbindung der Formel X-L-SO₂X
wobei
X für eine durch eine Aminogruppe substituierbare Abgangsgruppe wie beispielsweise Halogen steht;
L für eine Alkandiylgruppe oder eine Alkendiylgruppe mit jeweils 3 bis 5 Kohlenstoffatomen steht, wobei ein oder mehrere Kohlenstoffatome durch ein oder mehrere Heteroatome aus der Gruppe N, O, S ersetzt sein können, und wobei die Gruppe gegebenenfalls substituiert sein kann;
in Gegenwart einer organischen Base bei Raumtemperatur und anschließender Umsetzung mit einer Base in einem organischen Lösungsmittel unter Erhitzen.

5. Verbindungen der allgemeinen Formel (I) zur Behandlung von Krankheiten.

6. Arzneimittel enthaltend mindestens eine Verbindung der allgemeinen Formel (I) gemäß Anspruch 1.

7. Verfahren zur Herstellung von Arzneimitteln **dadurch gekennzeichnet, dass** man mindestens eine Verbindung der Formel (I) gemäß Anspruch 1, gegebenenfalls mit üblichen Hilfs- und Zusatzstoffen in eine geeignete Applikationsform überführt.

8. Arzneimittel enthaltend mindestens eine Verbindung der allgemeinen Formel (I) gemäß Anspruch 1 in Kombination mit organischen Nitraten oder NO-Donatoren.

9. Arzneimittel enthaltend mindestens eine Verbindung der allgemeinen Formel (I) gemäß Anspruch 1 in Kombination mit Verbindungen, die den Abbau von cyclischen Guanosinmonophosphat (cGMP) inhibieren.

10. Verwendung von Verbindungen der allgemeinen Formel (I) gemäß Anspruch 1 bei der Herstellung von Arzneimitteln zur Behandlung von Herz-Kreislauf-Erkrankungen.

11. Verwendung von Verbindungen der allgemeinen Formel (I) gemäß Anspruch 1 bei der Herstellung von Arzneimitteln zur Behandlung von Hypertonie.

12. Verwendung von Verbindungen der allgemeinen Formel (I) gemäß Anspruch 1 bei der Herstellung von Arzneimitteln zur Behandlung von thromboembolischen Erkrankungen und Ischämien.

13. Verwendung von Verbindungen der allgemeinen Formel (I) gemäß Anspruch 1 bei der Herstellung von Arzneimitteln zur Behandlung von sexueller Dysfunktion.

14. Verwendung von Verbindungen der allgemeinen Formel (I) gemäß Anspruch 1 bei der Herstellung von Arzneimitteln mit antiinflammatorischen Eigenschaften.

15. Verwendung von Verbindungen der allgemeinen Formel (I) gemäß Anspruch 1 bei der Herstellung von Arzneimitteln zur Behandlung von Erkrankungen des Zentralnervensystems.

16. Verwendung gemäß einem der Ansprüche 10 bis 15, wobei die Verbindungen der allgemeinen Formel gemäß Anspruch 1 in Kombination mit organischen Nitraten oder NO-Donatoren oder in Kombination mit Verbindungen, die den Abbau von cyclischen Guanosinmonophosphat (cGMP) inhibieren, eingesetzt werden.

## Claims

1. Compound of the formula (I) 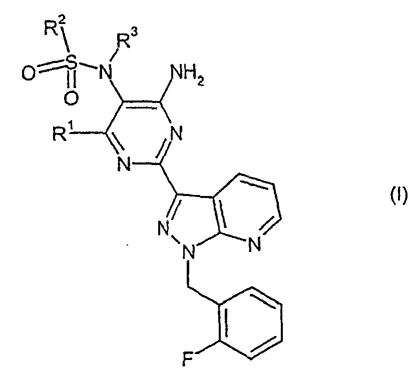 in which
R¹ is H, Cl or NH₂;
R² and R³ together with the heteroatoms to which they are bonded form a five- to seven-membered heterocycle which may be saturated or partially unsaturated, may optionally contain one or more other heteroatoms from the group of N, O, S, and may optionally be substituted;
and salts, isomers and hydrates thereof.

2. Compound according to Claim 1,
in which
R¹ is H, Cl or NH₂;
R² and R³ together with the heteroatoms to which they are bonded form a five- to seven-membered heterocycle which may be saturated or partially unsaturated, may optionally contain one or more other heteroatoms from the group of N, O, S;
and salts, isomers and hydrates thereof.

3. Compound according to Claim 1,
in which
R¹ is H, Cl or NH₂;
R² and R³ together with the heteroatoms to which they are bonded form a saturated five- to seven-membered heterocycle;
and salts, isomers and hydrates thereof.

4. Process for preparing compounds of the formula 1, comprising reaction of the compound of the formula (II) 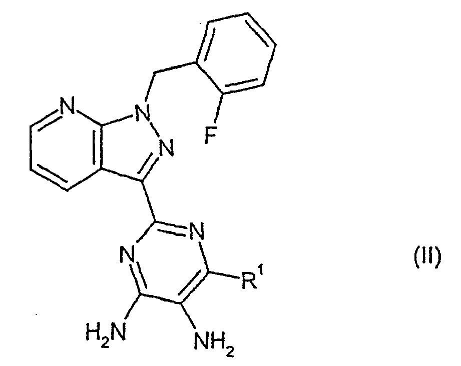 where R¹ is as defined above;
with a compound of the formula X-L-SO₂X
where
X is a leaving group which can be replaced by an amino group, such as, for example, halogen;
L is an alkanediyl group or an alkenediyl group having in each case 3 to 5 carbon atoms, where one or more carbon atoms may be replaced by one or more heteroatoms from the group of N, O, S, and where the group may optionally be substituted;
in the presence of an organic base at room temperature and subsequent reaction with a base in an organic solvent with heating.

5. Compound of the formula (I) for treating diseases.

6. Medicament comprising at least one compound of the formula (I) according to Claim 1.

7. Process for producing medicaments, **characterized in that** at least one compound of the formula (I) according to Claim 1 is converted into a suitable administration form, where appropriate with conventional excipients and additives.

8. Medicament comprising at least one compound of the formula (I) according to Claim 1 in combination with organic nitrates or NO donors.

9. Medicament comprising at least one compound of the formula (I) according to Claim 1 in combination with compounds which inhibit the breakdown of cyclic guanosine monophosphate (cGMP).

10. Use of compounds of the general formula (I) according to Claim 1 for producing medicaments for the treatment of cardiovascular disorders.

11. Use of compounds of the general formula (I) according to Claim 1 for producing medicaments for the treatment of hypertension.

12. Use of compounds of the general formula (I) according to Claim 1 for producing medicaments for the treatment of thromboembolic disorders and ischemias.

13. Use of compounds of the general formula (I) according to Claim 1 for producing medicaments for the treatment of sexual dysfunction.

14. Use of compounds of the general formula (I) according to Claim 1 for producing medicaments having antiinflammatory properties.

15. Use of compounds of the general formula (I) according to Claim 1 for producing medicaments for the treatment of disorders of the central nervous system.

16. Use according to any of Claims 10 to 15, where the compounds of the general formula according to claim 1 are employed in combination with organic nitrates or NO donors or in combination with compounds which inhibit the breakdown of cyclic guanosine monophosphate (cGMP).

## Revendications

1. Composés de formule générale (I) 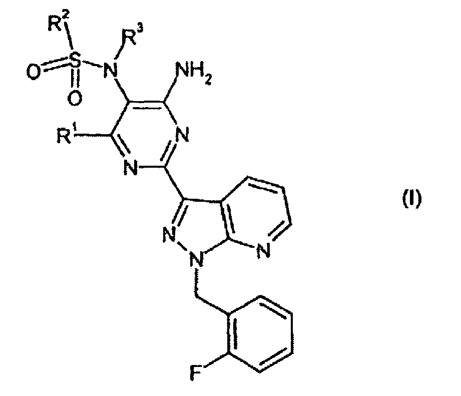 dans laquelle
R¹ représente H, Cl ou NH₂ ;
R² et R³ forment, conjointement avec les hétéroatomes auxquels ils sont liés, un hétérocycle penta- à heptagonal qui peut être saturé ou partiellement insaturé, qui peut contenir éventuellement un ou plusieurs autres hétéroatomes du groupe N, O, S et qui peut éventuellement être substitué ;
ainsi que leurs sels, leurs isomères et leurs hydrates.

2. Composés suivant la revendication 1,
dans lesquels
R¹ représente H, Cl ou NH₂ ;
R² et R³ forment, conjointement avec les hétéroatomes auxquels ils sont liés, un hétérocycle penta- à heptagonal qui peut être saturé ou partiellement insaturé, qui peut contenir éventuellement un ou plusieurs autres hétéroatomes du groupe N, O, S ;
ainsi que leurs sels, leurs isomères et leurs hydrates.

3. Composés suivant la revendication 1,
dans lesquels
R¹ représente H, Cl ou NH₂ ;
R² et R³ forment, conjointement avec les hétéroatomes auxquels ils sont liés, un hétérocycle saturé penta- à heptagonal ;
ainsi que leurs sels, leurs isomères et leurs hydrates.

4. Procédé de production de composés de formule 1 comprenant la réaction, en présence d'une base organique à la température ambiante, du composé de formule (II) 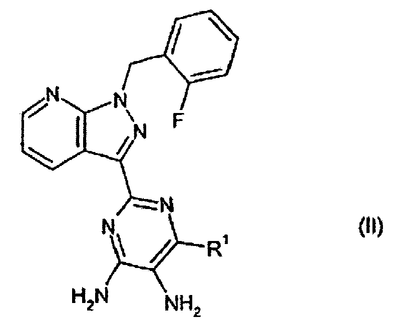 dans laquelle R¹ est tel que défini ci-dessus ;
avec un composé de formule X-L-SO₂X
dans laquelle
X représente un groupe partant pouvant être substitué par un groupe amino, tel que, par exemple, un halogène ;
L désigne un groupe alcanediyle ou un groupe alcènediyle ayant chacun 3 à 5 atomes de carbone, dont un ou plusieurs atomes de carbone peuvent être remplacés par un ou plusieurs hétéroatomes du groupe N, O, S, le groupe pouvant éventuellement être substitué ;
suivie de la réaction à la chaleur avec un base dans un solvant organique.

5. Composés de formule générale (I), destinés au traitement de maladies.

6. Médicament comprenant au moins un composé de formule générale (I) suivant la revendication 1.

7. Procédé de préparation de médicaments, **caractérisé en ce qu'**on fait prendre une forme d'administration convenable à au moins un composé de formule (I) suivant la revendication 1, éventuellement avec des substances auxiliaires et des additifs usuels.

8. Médicament contenant au moins un composé de formule générale (I) suivant la revendication 1, en combinaison avec des nitrates organiques ou des donneurs organiques de NO.

9. Médicament contenant au moins un composé de formule générale (I) suivant la revendication 1, en combinaison avec des composés qui inhibent la dégradation du guanosinemonophosphate cyclique (GMPc).

10. Utilisation de composés de formule générale (I) suivant la revendication 1, pour la préparation de médicaments destinés au traitement de maladies cardio-vasculaires.

11. Utilisation de composés de formule générale (I) suivant la revendication 1, pour la préparation de médicaments destinés au traitement de l'hypertonie.

12. Utilisation de composés de formule générale (I) suivant la revendication 1, pour la préparation de médicaments destinés au traitement de maladies thromboemboliques et d'ischémie.

13. Utilisation de composés de formule générale (I) suivant la revendication 1, pour la préparation de médicaments destinés au traitement d'un dysfonctionnement sexuel.

14. Utilisation de composés de formule générale (I) suivant la revendication 1, pour la préparation de médicaments possédant des propriétés anti-inflammatoires.

15. Utilisation de composés de formule générale (I) suivant la revendication 1, pour la préparation de médicaments destinés au traitement des maladies du système nerveux central.

16. Utilisation suivant l'une des revendications 10 à 15, dans laquelle les composés de formule générale suivant la revendication 1 sont utilisés en combinaison avec des nitrates organiques et des donneurs organiques de NO ou en combinaison avec des composés qui inhibent la dégradation du guanosinemonophosphate cyclique (GMPc).
